# EUROPEAN PATENT APPLICATION

(11) **EP 2 514 414 A1**
(43) Date of publication of application: **24.10.2012**
(21) Application number: 10837660.9
(22) Date of filing: 16.12.2010
(51) Int. Cl.: A61K 9/70, A61K 31/13, A61K 31/27, A61K 31/445, A61K 31/473, A61K 31/55, A61K 45/00, A61K 47/06, A61K 47/14, A61K 47/32, A61K 47/46, A61P 25/28

(54) **TRANSDERMALLY ABSORBED PREPARATION OF ANTI-DEMENTIA DRUG**

(30) Priority: 16.12.2009 JP 2009285450
(71) Applicant: Goto, Takeshi, Ushiku-shi, Ibaraki 300-1206 (JP)
(72) Inventor: ITO Takeshi, Fukuoka-shi Fukuoka 819-0043 (JP)
(74) Representative: Tetaz, Franck Claude Edouard
(86) International application number: PCT/JP2010/072666
(87) International publication number: WO 2011/074635

(57) **Abstract**

The present invention relates to a percutaneous absorption preparation that is lower skin irritation and enables efficient transdermal administration of an anti-dementia drug. More specifically, the present invention relates to a percutaneous absorption preparation comprising an anti-dementia drug, a polymer compound having an amino group, a polyvalent carboxylate ester, a fatty acid alkyl ester, a styrenic polymer compound, and a tackifier resin.

## Description

### [Cross-Reference to Related Application]

This application claims priority to Japanese Patent Application No. 2009-285450, filed on December 16, 2009, the entire disclosure of which is incorporated herein by reference.

### [Technical Field]

The present invention relates to a percutaneous absorption preparation comprising an anti-dementia drug, which is lower skin irritation.

### [Background Art]

In recent years, with increase in the number of elderly persons, the number of patients with dementia such as Alzheimer has also been increased and care for the patients and the like have become social problems. On the other hand, the development of anti-dementia drugs has also rapidly been pursued and, for example, donepezil hydrochloride has widely been used as a therapeutic agent for Alzheimer's disease, having an acetylcholinesterase inhibitory action. These anti-dementia drugs have been often orally administered via tablets and the like. As methods for administering drugs to patients, oral administration of tablets, capsules, syrups, granules, and the like as well as injection administration, rectal administration, and the like have been known and appropriately selected depending on the diseases of the patients and the features of the drugs.

However, a patient with an advanced symptom of dementia often gets into difficulty in taking an anti-dementia drug. Accordingly, the transdermal administration of the anti-dementia drug is considered to enable the continuous administration of the drug to the patient with the advanced symptom for a long period with avoiding the difficulty in taking it and to be particularly useful.

However, it is said that it is difficult to absorb a drug, of which the amount is effective for a sufficient effect, into the body through the skin since the skin generally has low drug permeability. To overcome such difficulty, a percutaneous absorption preparation comprising an anti-dementia drug has conventionally been studied.

For example, Japanese Patent Laid-Open No. 11-315016 (Patent Literature 1) discloses an ointment and the like for transdermal administration of an anti-dementia drug and a suppository for rectal administration, wherein the transdermal absorbability of donepezil hydrochloride is improved by a substrate comprising a higher alcohol and an ester derivative thereof.

In addition, WO 03/032960 (Patent Literature 2) discloses a transdermal absorption-type anti-dementia preparation comprising an adhesion composition, wherein the adhesion composition contains a dispersed active ingredient, the active ingredient is released at a pharmacologically effective rate, and a skin permeation rate is at least 1.2 µg/cm² or more per hour. Further, in the examples, disclosed is a preparation comprising an adhesion composition, prepared by containing donepezil hydrochloride which is an active ingredient, a styrene-isoprene-styrene block copolymer which is a hydrophobic polymer, and sodium acetate which is an organic acid salt, wherein the size of the preparation for single-dose administration for 24 hours is 60 cm².

In addition, in a transdermal preparation, a drug which is its active ingredient needs to be retained without precipitating in the preparation and to be stably placed on the skin. Thus, in consideration of, e.g., improvement of these functions, percutaneous absorption preparations and materials thereof have been examined.

For example, Japanese Patent Laid-Open No. 10-182439 (Patent Literature 3) discloses an adhesion- and bonding agent for a skin or transdermal treatment system, the adhesion-bonding agent comprising a (meth)acrylate copolymer containing a tertiary or quaternary amino group, an acrylate- or (meth)acrylate polymer or copolymer containing an acidic group, and a softening agent. As such softening agents in the examples, triethyl citrate and acetyl triethyl citrate are disclosed.

In addition, WO 02/38139 (Patent Literature 4) discloses a percutaneous absorption preparation comprising a polymer compound having an amino group, a drug forming an acid addition salt, and carboxylic acid and/or a salt thereof.

In addition, Japanese Patent Laid-Open No. 4-117323 (Patent Literature 5) discloses a transdermally absorbable patch prepared by retaining a patch layer containing an adhesive base and a drug on a support, wherein the transdermally absorbable patch comprises a certain amount of the drug in acid addition form and a polymer that contains a certain amount of basic nitrogen and has no adhesiveness on the skin at room temperature.

In addition, WO 2007/129427 (Patent Literature 6) discloses a reservoir-type percutaneous absorption preparation comprising an anti-dementia drug, wherein the percutaneous absorption preparation comprises at least an adhesive layer, an interlayer, and a drug-containing layer in this order from the side to be applied to the skin. The drug-containing layer of the percutaneous absorption preparation comprises at least an anti-dementia drug, a polymer compound having an amino group, a polyhydric alcohol, and one or more carboxylate esters and a period of applying the preparation is reported to be able to be set to be a long period even in the case of single-dose administration.

However, in percutaneous absorption preparations, maintenance of safety for the skin of a patient is a precondition for treatment. Accordingly, in a percutaneous absorption preparation comprising an anti-dementia drug, it is still demanded to reduce irritation to the skin of a patient while efficiently transdermally administering the drug.

### [Citation List]

### [Patent Literature]

[Patent Literature 1]
   Japanese Patent Laid-Open No. 11-315016
[Patent Literature 2]
   WO 03/032960
[Patent Literature 3]
   Japanese Patent Laid-Open No. 10-182439
[Patent Literature 4]
   WO 02/38139
[Patent Literature 5]
   Japanese Patent Laid-Open No. 4-117323
[Patent Literature 6]
   WO 2007/129427

### [SUMMARY OF INVENTION]

An object of the present invention is to provide a novel percutaneous absorption preparation that is lower skin irritation and enables efficient transdermal administration of an anti-dementia drug.

The percutaneous absorption preparation according to the present invention comprises a drug-containing layer comprising an anti-dementia drug, a polymer compound having an amino group, a polyvalent carboxylate ester, a fatty acid alkyl ester, a styrenic polymer compound, and a tackifier resin.

The percutaneous absorption preparation according to the present invention enables efficient transdermal administration of an anti-dementia drug while suppressing skin irritation.

### [BRIEF DESCRIPTION OF DRAWINGS]

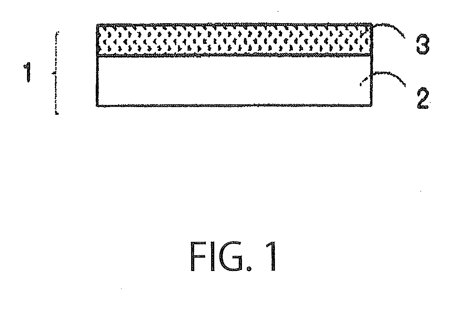

[Figure 1] Figure 1 illustrates an embodiment of a percutaneous absorption preparation according to the present invention.

### [DESCRIPTION OF EMBODIMENTS]

### Definition

As used herein, "alkyl" means straight-chain, branched, or cyclic alkyl, preferably having 2 to 18 carbon atoms in total.

In addition, as used herein, "alcohol" means a straight-chain, branched, or circular saturated or unsaturated alcohol.

### Percutaneous absorption preparation

The percutaneous absorption preparation according to the present invention is particularly advantageous for significantly effectively treating dementia while suppressing skin irritation when being used for being applied once daily. Accordingly, in accordance with a preferred embodiment of the present invention, the above-described percutaneous absorption preparation is used to be applied once daily.

The content of the anti-dementia drug is, without limitation, preferably 0.5-20 mass%, more preferably 3-20 mass%, further preferably 3-15 mass%, of the drug-containing layer in consideration of once daily application. Setting of the content of the anti-dementia drug in such a manner is preferred for efficient transdermal administration of the drug while suppressing skin irritation.

In addition, the anti-dementia drug according to the present invention is preferably a basic drug, more preferably a nitrogen-containing basic drug or a salt thereof, further preferably a nitrogen-containing basic drug.
Such salts of the anti-dementia drug as described above include, for example, hydrochlorides, tartrates, and hydrobromates, but are not limited thereto if being pharmaceutically acceptable salts.
More specifically, the anti-dementia drug is preferably donepezil, memantine, rivastigmine, galanthamine or tacrine, or salts thereof, more preferably donepezil or a salt thereof.

In addition, the polymer compound having an amino group in the drug-containing layer is preferably a copolymer constituted by a dialkylaminoalkyl (meth)acrylate and a monomer unit selected from alkyl (meth)acrylates, hydroxyalkyl (meth)acrylates, and combinations thereof. Such a copolymer is advantageous for stably retaining a drug and achieving a good drug flux.

Further, the polymer compound having an amino group is preferably an acryl (meth)acrylate-alkyl (meth)acrylate-dialkylaminoethyl (meth)acrylate copolymer, more preferably a copolymer comprising di-C₁₋₂-alkylamino-C₁₋₂-alkyl (meth)acrylate and C₁₋₄ alkyl (meth)acrylate and monohydroxy-C₂₋₄-alkyl (meth)acrylate as monomer units, further preferably a methyl (meth)acrylate-butyl (meth)acrylate-dimethylaminoethyl (meth)acrylate copolymer, further preferably a methyl methacrylate-butyl methacrylate-dimethylaminoethyl methacrylate copolymer. Such a methyl methacrylate-butyl methacrylate-dimethylaminoethyl methacrylate copolymer is commercially available, for example, as Eudragit^{®} E100 (Degussa Corporation).

In addition, in the above-described polymer compound having an amino group, the molar ratio, the molecular weight, and the like of a monomer unit may appropriately be regulated by the person skilled in the art.

In addition, the content of the polymer compound having an amino group in the drug-containing layer is, without particular limitation, preferably 1-25 mass%, more preferably 3-20 mass%.

The above-described polyvalent carboxylate ester is preferably a divalent to hexavalent carboxylate ester, a divalent to trivalent C₁₋₆ alkyl carboxylate ester, more preferably a divalent to trivalent C₁₋₆ alkyl carboxylate ester, further preferably a divalent to trivalent C₁₋₃ alkyl carboxylate ester, further preferably a tri-C₁₋₃-alkyl citrate ester or a di-C₁₋₃-alkyl sebacate ester, further preferably triethyl citrate or diethyl sebacate.

In addition, the content of the polyvalent carboxylate ester in the drug-containing layer is, without particular limitation, preferably 1-10 mass%, more preferably 2-5 mass%.

In addition, the fatty acid alkyl ester is preferably a C₆₋₁₈ fatty acid alkyl ester, a C₁₂₋₁₈-fatty acid-C₁₋₁₈-alkyl ester.
In addition, examples of the fatty acid alkyl ester include isopropyl myristate, methyl myristate, isopropyl palmitate, ethyl oleate, decyl oleate, oleyl oleate, methyl laurate, hexyl laurate, or the like, preferably isopropyl myristate, isopropyl palmitate, oleyl oleate, or hexyl laurate, more preferably isopropyl myristate.

In addition, the content of the fatty acid alkyl ester in the drug-containing layer is, without particular limitation, preferably 1-20 mass%, more preferably 3-15 mass%.

In addition, the styrenic polymer compound is, without particular limitation unless precluding the release and retention of the drug, preferably a copolymer of styrene with a polymerizable alkene having 2 to 8 carbon atoms, more preferably a styrene-isoprene-styrene block copolymer, styrene-butylene-styrene block copolymer, or a styrene-butadiene-styrene block copolymer, further preferably a styrene-isoprene-styrene block copolymer. Such a styrene-isoprene-styrene block copolymer is, for example, Kraton^{®} D1111K (manufactured by Kraton Corporation).

In addition, the content of the styrenic polymer compound in the drug-containing layer is, without particular limitation, preferably 5-90 mass%, more preferably 10-80 mass%.

In addition, the tackifier resin is preferably at least one resin selected from the group consisting of an alicyclic saturated hydrocarbon resin, a rosin resin, a hydrogenated rosin resin, a rosin ester resin and a hydrogenated rosin ester resin and a terpene resin, more preferably an alicyclic saturated hydrocarbon resin, a rosin, a rosin glycerol ester, a hydrogenated rosin, and a hydrogenated rosin glycerol ester, further preferably an alicyclic saturated hydrocarbon resin. Such an alicyclic saturated hydrocarbon resin is commercially available, for example, as Arkon P-100^{®} (manufactured by Arakawa Chemical Industries, Ltd.).

In addition, the content of the tackifier resin in the drug-containing layer is, without particular limitation, preferably 10-80 mass%, more preferably 15-70 mass%.

In addition, the percutaneous absorption preparation according to the present invention may be constituted by the above-described constituents but preferably further comprises an additive selected from a polyhydric alcohol fatty acid ester and a liquid paraffin.

The polyhydric alcohol fatty acid ester is preferably a sugar-alcohol fatty acid ester or a glycol fatty acid ester, more preferably a sugar-alcohol fatty acid ester, further preferably at least one selected from the group consisting of sorbitan fatty acid ester, propylene glycol fatty acid ester, and glycerol fatty acid ester, further preferably sorbitan fatty acid ester.

As more specific examples, the polyhydric alcohol fatty acid esters include sorbitan monolaurate, sorbitan monostearate, sorbitan monooleate, sorbitan monopalmitate, sorbitan trioleate, or sorbitan tristearate, preferably sorbitan monolaurate.

In addition, the content of the polyhydric alcohol fatty acid ester or the liquid paraffin in the drug-containing layer is, without particular limitation, preferably 3-30 mass%, more preferably 5-20 mass%.

### Combination

The drug-containing layer according to the present invention may be formed by appropriately combining such components as described above as far as the components and the amounts thereof are used.
In accordance with a preferred embodiment of the present invention, in the above-described percutaneous absorption preparation, the anti-dementia drug is a basic anti-dementia drug or a salt thereof and the polymer compound having an amino group is a copolymer constituted by a dialkylaminoalkyl (meth)acrylate and a monomer unit selected from an alkyl (meth)acrylate, a hydroxyalkyl (meth)acrylates, and a combinations thereof.

In addition, in accordance with a further preferred embodiment of the present invention, in the above-described percutaneous absorption preparation, the anti-dementia drug is a basic anti-dementia drug or a salt thereof, the polymer compound having an amino group is a copolymer constituted by a dialkylaminoalkyl (meth)acrylate and a monomer unit selected from an alkyl (meth)acrylate, a hydroxyalkyl (meth)acrylate, and a combination thereof, and the polyvalent carboxylate ester is a divalent to hexavalent carboxylate ester.

In addition, in accordance with a further preferred embodiment of the present invention, in the above-described percutaneous absorption preparation, the anti-dementia drug is a basic anti-dementia drug or a salt thereof, the polymer compound having an amino group is a copolymer constituted by a dialkylaminoalkyl (meth)acrylate and a monomer unit selected from an alkyl (meth)acrylate, a hydroxyalkyl (meth)acrylate, and a combination thereof, the polyvalent carboxylate ester is a divalent to hexavalent carboxylate ester, and the fatty acid alkyl ester is a C₆₋₁₈ fatty acid alkyl ester.

In addition, in accordance with a further preferred embodiment of the present invention, in the above-described percutaneous absorption preparation, the anti-dementia drug is a basic anti-dementia drug or a salt thereof, the polymer compound having an amino group is a copolymer constituted by a dialkylaminoalkyl (meth)acrylate and a monomer unit selected from an alkyl (meth)acrylate, a hydroxyalkyl (meth)acrylate, and a combination thereof, the polyvalent carboxylate ester is a divalent to hexavalent carboxylate ester, the fatty acid alkyl ester is a C₆₋₁₈ fatty acid alkyl ester, and the styrenic polymer compound is a copolymer of styrene with a polymerizable alkene having 2 to 8 carbon atoms.

In addition, in accordance with a further preferred embodiment of the present invention, in the above-described percutaneous absorption preparation, the anti-dementia drug is a basic anti-dementia drug or a salt thereof, the polymer compound having an amino group is a copolymer constituted by a dialkylaminoalkyl (meth)acrylate and a monomer unit selected from an alkyl (meth)acrylate, a hydroxyalkyl (meth)acrylate, and a combination thereof, the polyvalent carboxylate ester is a divalent to hexavalent carboxylate ester, the fatty acid alkyl ester is a C₆₋₁₈ fatty acid alkyl ester, the styrenic polymer compound is a copolymer of styrene with a polymerizable alkene having 2 to 8 carbon atoms, and the tackifier resin is at least one resin selected from an alicyclic saturated hydrocarbon resin, a rosin resin, a hydrogenated rosin resin, a rosin ester resin, and a hydrogenated rosin ester resin.

In addition, in any of the above-described preferred embodiments, the percutaneous absorption preparation preferably further includes an additive selected from polyhydric alcohol fatty acid esters and liquid paraffins.
In addition, in any of the above-described preferred embodiments, the basic anti-dementia drug or the salt thereof is preferably donepezil, memantine, rivastigmine, galanthamine or tacrine, or salts thereof, more preferably donepezil or a salt thereof.
In addition, in any of the above-described preferred embodiments, the polymer compound having an amino group is preferably an acryl (meth)acrylate-alkyl (meth)acrylate-dialkylaminoethyl (meth)acrylate copolymer, more preferably a methyl methacrylate-butyl methacrylate-dimethylaminoethyl methacrylate copolymer.

In addition, in any of the above-described preferred embodiments, the polyvalent carboxylate ester is preferably a divalent to hexavalent carboxylate ester, more preferably a divalent to trivalent C₁₋₆ alkyl carboxylate ester, further preferably a sebacate ester or a citrate ester.

In addition, in any of the above-described preferred embodiments, the fatty acid alkyl ester is preferably a C₆₋₁₈ fatty acid alkyl ester, more preferably a C₁₂₋₁₈-fatty acid-C₁₋₁₈-alkyl ester, further preferably isopropyl myristate.

In addition, in any of the above-described preferred embodiments, the styrenic polymer compound is preferably a copolymer of styrene with a polymerizable alkene having 2 to 8 carbon atoms, more preferably a styrene-isoprene-styrene block copolymer, a styrene-butylene-styrene block copolymer, or a styrene-butadiene-styrene block copolymer, further preferably a styrene-isoprene-styrene block copolymer.

In addition, in any of the above-described preferred embodiments, the tackifier resin is preferably an alicyclic saturated hydrocarbon resin, rosin, rosin glycerol ester, hydrogenated rosin, and hydrogenated rosin glycerol ester, more preferably an alicyclic saturated hydrocarbon resin.

In addition, in any of the above-described preferred embodiments, the polyhydric alcohol fatty acid ester is preferably a sugar-alcohol fatty acid ester, more preferably sorbitan fatty acid ester.

In addition, the thickness of the drug-containing layer according to the present invention, which thickness is appropriately determined in consideration of a drug amount by the person skilled in the art, may be, for example, 30-150 µm.

The drug-containing layer of the percutaneous absorption preparation according to the present invention may also be utilized as a percutaneous absorption preparation without being processed and may be constituted by appropriately containing a support and/or a cover as well as the drug-containing layer.
Figure 1 is a cross-sectional view illustrating one embodiment of the percutaneous absorption preparation according to the present invention.
In Figure 1, a percutaneous absorption preparation 1 according to the present invention is constituted by a drug-containing layer 2 and a support 3 in this order from the skin side. The drug-containing layer 2 is placed on a skin-contacting surface and can adhere the percutaneous absorption preparation 1 to the skin.

The support may be elastic or non-elastic. Specific materials constituting the support include, but are not particularly limited to, for example, woven fabrics, non-woven fabrics, PET (polyethylene terephthalate), polyurethane, polyester, polyethylene, composite materials thereof, or the like.
In addition, as the materials of the cover, which are not particularly limited, the same materials as those of the support may be used.

### Production Method

A preferred method for producing the percutaneous absorption preparation according to the present invention is as follows.
First, the components of the drug-containing layer according to the present invention are appropriately mixed in a solvent to adjust a liquid mixture containing the components in the solvent. Then, the liquid mixture is used as a plaster solution and applied onto a liner. Then, the plaster solution is dried at around 60-120°C to obtain the drug-containing layer and, as needed, a support is laminated thereon to obtain a percutaneous absorption preparation.
In addition, when a cover is mounted on the percutaneous absorption preparation, for example, the cover, on one surface of which an adhesive layer is placed, may also be prepared to affix the one surface of the adhesive layer side of the cover and one surface of the support side of the percutaneous absorption preparation to each other.

In the above-described production method, examples of the solvent which is used when the drug-containing layer and the adhesive layer are prepared include ethyl acetate, butyl acetate, toluene, n-hexane, n-heptane, tetrahydrofuran, dimethylformamide, methanol, ethanol, or the like.

In addition, the percutaneous absorption preparation according to the present invention is preferably applied around once daily in consideration of the suppression of skin irritation and the stable and efficient administration of a drug, as described above. Accordingly, in accordance with another embodiment of the invention, provided is a method for treating dementia, comprising applying the above-described percutaneous absorption preparation to the skin of an organism once daily.

In addition, such organisms as described above include mammals, preferably human.

### [Examples]

The present invention will be specifically described below with reference to Examples but is not limited to these Examples.

### Example 1

### Preparation of Drug-containing Layer

Donepezil, Eudragit^{®} E100, triethyl citrate, IPM (isopropyl myristate), and SML (sorbitan monolaurate) were prepared in the the-described formulation ratio and mixed and stirred in an appropriate amount of toluene. SIS (styrene-isoprene-styrene block copolymer, Kraton^{®} D1111K, manufactured by Kraton Corporation) and Arkon P-100 (manufactured by Arakawa Chemical Industries, Ltd.) were added in a formulation ratio as described below to the obtained liquid mixture to obtain a plaster solution.

**[Table 1]**

| Constituent | mass% |
|---|---|
| Donepezil | 5 |
| Eudragit^{®} E100 | 3.53 |
| Triethyl citrate | 10 |
| IPM | 15 |
| SML | 5 |
| SIS | 21.15 |
| Arkon P-100 | 45.32 |

The above-described plaster solution was applied onto a liner made of polyethylene terephthalate, dried at 80°C for 15 minutes to obtain a drug-containing layer, and the amount of the drug-containing layer after the drying was adjusted to be 50 g_{/}m².
Then, a support (Scotchpak^{™} 9732, manufactured by 3M Company) was laminated opposite side of the liner of the drug-containing layer to obtain a percutaneous absorption preparation.

### Example 2

A percutaneous absorption preparation was prepared by the same procedure as in Example 1 except that the formulation was changed as described below.

**[Table 2]**

| Constituent | mass% |
|---|---|
| Donepezil | 5 |
| Eudragit^{®} E100 | 3.53 |
| Triethyl citrate | 10 |
| IPM | 10 |
| Liquid paraffin | 5 |
| SIS | 21.15 |
| Arkon P-100 | 45.73 |

### Comparative Example 1: Drug-containing Layer Containing 25 mass% of Donepezil Hydrochloride

A percutaneous absorption preparation was prepared by the same procedure as in Example 1 except that the formulation in the drug-containing layer was changed as described below.

**[Table 3]**

| Constituent | mass% |
|---|---|
| Donepezil hydrochloride | 25 |
| Eudraqit^{©} E100 | 17.67 |
| Triethyl citrate | 10 |
| Glycerol | 10 |
| SML | 5 |
| SIS | 32.33 |

### Test Example 1

The percutaneous absorption preparations (20 mm in diameter) were applied to the backs of rabbits (Japanese white species, male, 16-23-week-old, n = 6) for 24 hours. Skin irritation indices for erythema and edema were calculated with reference to Draize skin irritation evaluation criteria and an evaluation procedure therefor 1, 24, and 48 h after removal of the preparations.

**[Table 4] Draize Skin Irritation Evaluation Criteria**

| Evaluation Criteria for Skin Reaction | Score |
|---|---|
| Erythema and eschar formation | |
| No erythema | 0 |
| Very slight erythema (barely perceptible) | 1 |
| Well defined erythema | 2 |
| Moderate to severe erythema | 3 |
| Severe erythema (beet redness) to slight eschar formation (injuries in depth) | 4 |
| Edema | |
| No edema | 0 |
| Very slight edema (barely perceptible) | 1 |
| Slight edema (edges of area well defined by definite raising) | 2 |
| Moderate edema (raised approximately 1 mm) | 3 |
| Severe edema (raised more than 1 mm extending beyond area of exposure) | 4 |

The results are listed in Table 5.

**[Table 5]**

| Preparation | Skin irritation index* |
|---|---|
| Example 1 | 1.06 |
| Example 2 | 1.04 |
| Comparative Example 1 | 1.72 |

| | |
|---|---|
| *Abraded skin was not evaluated. | |

The skin irritations in Examples 1 and 2 were confirmed to be lower than that in Comparative Example 1.

### Test Example 2

*In Vitro* Hairless Mouse Dermal Penetration Test (24 hours)
The percutaneous absorption preparation (application area of 2.5 cm²) of Example 1 or Comparative Example 1 was applied to the stratum corneum layer side of the hairless mouse skin and set in a flow-through cell (5 cm²) through which warm water was circulated so that a skin surface was at about 32°C. A phosphate buffered saline solution (pH 7.4) was used as a receiver phase and the receiver phase was sampled at a rate of 2.5 mL/hr every 4 hours until 24 hours. For the sampled solution, a drug amount was measured by HPLC, a penetration rate per hour was calculated, and the mean value ± standard deviation of a flux (mcg/cm²/hr) per unit area was determined every 4 hours.

In addition, as a result of calculating dermal penetration amounts from the sampled solutions, the dermal penetration amount of Example 1 was 233.8 ± 2.7 (mcg/cm²) and the dermal penetration amount of Example 2 was 233.1 ± 8.8 (mcg/cm²). In contrast, the dermal penetration amount of Comparative Example 1 was 463.2 ± 52.0 (mcg/cm²).

In addition, as a result of calculating the residual drug amount rates of Examples 1 and 2 and Comparative Example 1 in the preparations based on the dermal penetration amounts, the rate of Example 1 was 4.6-8.4% and the rate of Example 2 was 1.6-13.5%.
In contrast, the residual drug amount rate of Comparative Example 1 (containing 25 mass% of the drug) was 54.6-67.2%.
The residual drug amount rates of Examples 1 and 2 were lower than that of Comparative Example 1, so that the drug was confirmed to be efficiently absorbed.

## Claims

1. A percutaneous absorption preparation comprising an anti-dementia drug, a polymer compound having an amino group, a polyvalent carboxylate ester, a fatty acid alkyl ester, a styrenic polymer compound, and a tackifier resin.

2. The percutaneous absorption preparation according to claim 1, which is used to be applied to human once daily, wherein the content of the anti-dementia drug is 0.5-20 mass% of the drug-containing layer.

3. The percutaneous absorption preparation according to claim 1 or 2, further comprising an additive selected from a polyhydric alcohol fatty acid ester and a liquid paraffin.

4. The percutaneous absorption preparation according to any one of claims 1 to 3, wherein the anti-dementia drug is a basic drug or a salt thereof.

5. The percutaneous absorption preparation according to any one of claims 1 to 4, wherein the anti-dementia drug is donepezil, memantine, rivastigmine, galanthamine, tacrine, or salts thereof.

6. The percutaneous absorption preparation according to any one of claims 1 to 5, wherein the polymer compound having an amino group is a copolymer composed of a dialkylaminoalkyl (meth)acrylate and a monomer unit selected from an alkyl (meth)acrylate, a hydroxyalkyl (meth)acrylate, and a combination thereof.

7. The percutaneous absorption preparation according to any one of claims 1 to 6, wherein the polymer compound having an amino group is an acryl (meth)acrylate-alkyl (meth)acrylate-dialkylaminoethyl (meth)acrylate copolymer.

8. The percutaneous absorption preparation according to any one of claims 1 to 6, wherein the polyvalent carboxylate ester is a divalent to hexavalent carboxylate ester.

9. The percutaneous absorption preparation according to any one of claims 1 to 8, wherein the polyvalent carboxylate ester is a sebacate ester or a citrate ester.

10. The percutaneous absorption preparation according to any one of claims 1 to 9, wherein the fatty acid alkyl ester is a C₆₋₁₈ fatty acid alkyl ester.

11. The percutaneous absorption preparation according to any one of claims 1 to 10, wherein the styrenic polymer compound is a copolymer of styrene with polymerizable alkene having 2 to 8 carbon atoms.

12. The percutaneous absorption preparation according to any one of claims 1 to 11, wherein the styrenic polymer compound is a styrene-isoprene-styrene block copolymer, a styrene-butylene-styrene block copolymer, or a styrene-butadiene-styrene block copolymer.

13. The percutaneous absorption preparation according to any one of claims 1 to 12, wherein the tackifier resin is at least one resin selected from an alicyclic saturated hydrocarbon resin, a rosin resin, a hydrogenated rosin resin, a rosin ester resin, and a hydrogenated rosin ester resin.

14. The percutaneous absorption preparation according to any one of claims 3 to 13, wherein the polyhydric alcohol fatty acid ester is a sugar-alcohol fatty acid ester or a glycol fatty acid ester.
